# EUROPEAN PATENT APPLICATION

(11) **EP 3 936 072 A1**
(43) Date of publication of application: **12.01.2022**
(21) Application number: 21182327.3
(22) Date of filing: 29.06.2021
(51) Int. Cl.: A61B 18/22, A61B 18/00, A61B 1/00, A61B 8/12, A61B 18/26, A61B 17/00, A61B 1/015, A61B 1/018

(54) **AUTONOMOUS ULTRASOUND GUIDED ENDOSCOPE**

(30) Priority: 08.07.2020 US 202063049166 P; 02.06.2021 US 202117337257
(71) Applicant: Covidien LP, Boulder, Colorado 80301-3299 (US)
(72) Inventor: SARTOR, Joe D., Boulder, 80301 (US); HEARD, David N., Boulder, 80301 (US); FRUSHOUR, Scott, Boulder, 80301 (US); GLEIMAN, Seth S., North Haven, 06473 (US)
(74) Representative: Viering, Jentschura & Partner mbB Patent- und Rechtsanwälte

(57) **Abstract**

A surgical system includes an endoscope, a computing device, a vacuum source, and a laser source. The endoscope defines an elongate body having an ultrasound sensor disposed on a distal portion thereof, a first channel defined through the elongate body defining a first aperture extending through the distal portion, a second channel defined through the elongate body defining a second aperture extending through the distal portion and having an optical waveguide disposed therein. The computing device includes a display screen and a processor having a memory storing a software application, which when executed, processes image data captured by the ultrasound sensor and displays a representation of the patient's anatomy on the display screen. The vacuum source is in fluid communication with the first channel to cause a stone to be retained against the distal portion of the endoscope. The laser source is in electromagnetic communication with the optical waveguide.

## Description

### CROSS-REFERENCE TO RELATED APPLICATION

This application claims the benefit of and priority to U.S. Provisional Patent Application No. 63/049,166, filed on July 8, 2020, the entire disclosure of which is incorporated by reference herein.

### TECHNICAL FIELD

The disclosure relates generally to surgical systems, and more particularly, to systems and methods for treating kidney and bladder stones using an autonomous ultrasound guided endoscope.

### BACKGROUND

Laser lithotripsy is a widely utilized tool for treating kidney and bladder stones. Typically, during a procedure, the kidney stone is visualized through a flexible optical endoscope. Identifying kidney stones which are located deeper in the kidney than the patient's renal pelvis can be difficult due to the tortuosity of the calyx. Removal of the kidney during laser lithotripsy requires the laser to be tuned at a frequency that is highly absorbed by fluid, such as water or saline, in an effort to avoid overshooting the kidney stone and inadvertently damaging surrounding tissue. As can be appreciated, the high absorption rate of the laser energy in the fluid requires the output of the laser to be in direct contact with the kidney stone. As such, visualizing the kidney stone using the flexible optical endoscope requires the flexible optical endoscope to be offset from the kidney stone (*e.g.,* in a standoff position relative to the kidney stone). Therefore, to place the output of the laser in direct contact with the kidney stone, the laser optical waveguide or fiber must be extended from the flexible optical endoscope. Care must be taken when extending the laser fiber to contact the kidney stone to ensure that the kidney stone is not moved out of position.

During treatment, the laser is activated in either a large energy pulse, which is intended to fracture the kidney stone, or in small energy pulses, which are intended to erode the surface of the kidney stone (*e.g.,* dust the stone). In procedures utilizing small energy pulses, as the surface of the kidney stone is eroded, contact with the surface of the kidney stone may be lost. To alleviate this issue, suction or other mechanical means are utilized to maintain the appropriate spacing between the surface of the kidney stone and the flexible optical endoscope while also maintaining contact between the kidney stone and the laser fiber.

In a related procedure, prostate hyperplasia is similarly treated with an endoscope. Prostate hyperplasia is commonly treated via a process called transurethral resection of the prostate (TURP) or often using transurethral holmium laser enucleation of the prostate (HoLEP). During a TURP procedure, an electrosurgical loop electrode is utilized to shave slivers of the patient's internal prostate under direct visualization by the clinician and to provide hemostasis. As can be appreciated, removing adequate amounts of tissue from the patient's internal prostate in a timely fashion is challenging due to bleeding the slow resection speeds. In an effort to alleviate this issue, HoLEP attempts to excise the entire prostate transition zone in large chunks under direct visualization by the clinician using blunt dissection and using laser coagulation to provide hemostasis as needed. Further, HoLEP manually morcellates the large chunks of internal prostate tissue for transurethral removal. HoLEP removes more tissue than TURP and is therefore more efficacious, however, it is more technically challenging for the clinician to visualize the correct tissue planes and remove adequate amounts of internal prostate tissue in a timely manner. The 5-year risk rate of TURP for reoperation is approximately 5-10%, whereas many clinicians consider HoLEP to be a lifetime procedure due to more complete removal of prostate transitional zone tissue.

### SUMMARY

The disclosure relates to a surgical system including an endoscope, a computing device, a vacuum source, and a laser source. The endoscope defines an elongate body and includes an ultrasound sensor disposed on a distal portion of the elongate body, a first channel defined through the elongate body of the endoscope defining a first aperture extending through the distal portion of the elongate body, a second channel defined through the elongate body and defining a second aperture extending through the distal portion of the elongate body, and an optical waveguide disposed within the second channel. The computing device includes a display screen and a processor including a memory associated therewith for storing a software application, which when executed, processes image data captured by the ultrasound sensor and displays a representation of a patient's anatomy on the display screen. The vacuum source is in fluid communication with the first channel and is configured to cause a stone of a patient to be retained against the distal portion of the endoscope. The laser source is in electromagnetic communication with the optical waveguide.

In aspects, the ultrasound sensor may be a capacitive micromachined ultrasonic transducer.

In certain aspects, the ultrasound sensor may be a piezoelectric micromachined ultrasonic transducer.

In other aspects, the surgical system may include a lens disposed on the distal portion of the elongate body of the endoscope and in electromagnetic communication with the optical waveguide.

In certain aspects, the lens may define a bevel thereon that is configured to direct propagation of energy generated by the laser at an angle relative to a longitudinal axis defined by the elongate body of the endoscope.

In aspects, the optical waveguide may be rotatably supported within the second channel, wherein rotation of the optical waveguide relative to the lens causes the direction in which energy generated by the laser propagates therefrom.

In other aspects, the lens may be rotatably supported on the distal portion of the elongate body of the endoscope, wherein rotation of the lens relative to the optical waveguide causes the direction in which energy generated by the laser propagates therefrom.

In aspects, a portion of the first channel may define a protrusion thereon that is configured to collect fragments of a stone of a patient.

In certain aspects, the protrusion may be an annular boss disposed on an inner surface of the first channel.

In other aspects, the protrusion may be a protuberance disposed on an inner surface of the first channel.

In accordance with another aspect of the disclosure, an endoscope is provided including an elongate body defining a distal portion, an ultrasound sensor disposed on the distal portion of the elongate body, a first channel defined through the elongate body and defining a first aperture extending through the distal portion of the elongate body, a second channel defined through the elongate body and defining a second aperture extending through the distal portion of the elongate body, an optical waveguide disposed within the second channel and in electromagnetic communication with the laser, and a lens disposed on the distal portion of the elongate body and in electromagnetic communication with the optical waveguide, the lens configured to alter the direction in which energy is generated by the laser propagates relative to the distal portion of the elongate body.

In aspects, the first channel may be in fluid communication with a vacuum source that is configured to cause a stone of a patient to be retained against the distal portion of the elongate body.

In certain aspects, the ultrasound sensor may be a capacitive micromachined ultrasonic transducer.

In other aspects, the ultrasound sensor may be a piezoelectric micromachined ultrasonic transducer.

In certain aspects, the lens may define a bevel thereon that is configured to direct propagation of energy generated by the laser at an angle relative to a longitudinal axis defined by the elongate body of the endoscope.

In aspects, the optical waveguide may be rotatably supported within the second channel, wherein rotation of the optical waveguide relative to the lens causes the direction in which energy generated by the laser propagates therefrom.

In other aspects, the lens may be rotatably supported on the distal portion of the elongate body, wherein rotation of the lens relative to the optical waveguide causes the direction in which energy generated by the laser propagates therefrom.

In aspects, a portion of the first channel may define a protrusion thereon that is configured to collect fragments of a stone of a patient.

In certain aspects, the protrusion may be an annular boss disposed on an inner surface of the first channel.

In other aspects, the protrusion may be a protuberance disposed on an inner surface of the first channel.

In accordance with another aspect of the disclosure a surgical system is provided including an endoscope and a computing device. The endoscope defines an elongate body and includes an ultrasound sensor disposed on a distal portion of the elongate body, a channel defined through the elongate body defining an aperture extending through the distal portion of the elongate body, and a shaver slidably disposed within the channel and extendable from the aperture. The computing device includes a display screen and a processor having a memory associated therewith for storing a software application, which when executed, processes image data captured by the ultrasound sensor and displays a representation of a patient's anatomy on the display screen.

In aspects, the ultrasound sensor may be a capacitive micromachined ultrasonic transducer.

In other aspects, the ultrasound sensor may be a piezoelectric micromachined ultrasonic transducer.

In certain aspects, the shaver may be a rotary shaver that is slidably and rotatably supported within the channel.

In other aspects, the shaver may be a reciprocal shaver that is slidably and rotatably supported within the channel.

In certain aspects, the shaver may define a throughbore that is in fluid communication with an aperture defined through a distal portion of the shaver.

In aspects, the aperture of the shaver may define a sharpened edge that is configured to sever tissue.

In certain aspects, the surgical system may include a vacuum source that is in fluid communication with the throughbore of the shaver.

In aspects, the representation of the patient's anatomy may be a two-dimensional representation of the patient's anatomy.

In other aspects, the representation of the patient's anatomy may be a three-dimensional representation of the patient's anatomy.

In accordance with another aspect to the disclosure, an endoscope is provided including an elongate body defining a distal portion, an ultrasound sensor disposed on the distal portion of the elongate body, a channel defined through the elongate body, and a shaver slidably disposed within the channel and extendable from the aperture.

In aspects, the ultrasound sensor may be a capacitive micromachined ultrasonic transducer.

In certain aspects, the ultrasound sensor may be a piezoelectric micromachined ultrasonic transducer.

In other aspects, the shaver may be a rotary shaver that is slidably and rotatably supported within the channel.

In certain aspects, the shaver may be a reciprocal shaver that is slidably and rotatably supported within the channel.

In aspects, the shaver may define a throughbore that is in fluid communication with an aperture defined through a distal portion of the shaver.

In other aspects, the aperture of the shaver may define a sharpened edge that is configured to sever tissue.

In certain aspects, the endoscope may include a vacuum source in fluid communication with the throughbore of the shaver.

In other aspects, the throughbore of the shaver may be in fluid communication with a vacuum source.

In certain aspects, the aperture of the shaver may define a plurality of sharpened edges configured to sever tissue.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiments given below, serve to explain the principles of the disclosure, wherein:
FIG. 1 is a schematic view of a surgical system provided in accordance with the disclosure including an ultrasound guided endoscope;
FIG. 2 is a perspective view of the ultrasound guided endoscope of FIG. 1;
FIG. 2A is an enlarged view of the area of detail indicated on FIG. 2;
FIG. 3 is a perspective view of the ultrasound guided endoscope of FIG. 1;
FIG. 3A is a side, cross-sectional, view of the ultrasound guided endoscope taken along section line 3A-3A of FIG. 3;
Fig. 4 is a side view of the ultrasound guided endoscope of FIG. 1 illustrating a movable optical waveguide disposed therein;
FIG. 5 is a side view of the ultrasound guided endoscope of FIG. 1 illustrated a fixed optical waveguide disposed therein;
FIG. 6 is a side, cross-sectional view of the ultrasound guided endoscope of FIG. 5;
FIG. 7 is a side view of the ultrasound guided endoscope of FIG. 1 illustrating a stationary portion and an extendible portion of the ultrasound guided endoscope;
FIG. 8 is a side view of the ultrasound guided endoscope of FIG. 1 illustrating a distal manipulatable segment of the ultrasound guided endoscope;
FIG. 9 is a perspective view of another embodiment of an ultrasound guided endoscope provided in accordance with the disclosure;
FIG. 10 is a side view of a shaver of the ultrasound guided endoscope of FIG. 9;
FIG. 11 is a schematic illustration of a robotic surgical system configured for use in accordance with the disclosure; and
FIG. 12 is a schematic illustration of a surgical system configured for lung navigation in accordance with the disclosure.

### DETAILED DESCRIPTION

The disclosure is directed to surgical systems including ultrasound guided endoscopes. As described herein, the surgical systems including autonomous ultrasound guided endoscopes include a computing device and an endoscope for navigating to and treating a kidney stone of a patient. The endoscope includes a distal portion having an ultrasound sensor disposed thereon for generating a two-dimensional or three-dimensional representation of the patient's anatomy distal of the distal portion of the endoscope. It is envisioned that the ultrasound sensor may be a miniaturized ultrasound sensor, such as a capacitive micromachined ultrasonic transducer or a piezoelectric micromachined ultrasonic transducer. The use of the ultrasound sensor during cystoscopy or ureteroscopy (*e.g*., procedures in which the endoscope is submerged in fluid) enables a clinician to observe a clearer image of the patient's anatomy distal of the endoscope, and therefore, enables a clinician to more easily navigate the patient's luminal network in addition to more accurately visualizing the kidney or bladder stone during treatment. Specifically, use of the ultrasound sensor permits the endoscope to be in contact with the kidney or bladder stone during treatment, rather than maintaining a gap as is required using optical endoscopes.

The distal portion of the endoscope also includes a first aperture defined therein that is in fluid communication with a first channel defined within an elongate body of the endoscope. The first channel is in fluid communication with a fluid source and/or a vacuum source. As can be appreciated, the vacuum source provides suction at the first aperture to cause the kidney or bladder stone to be held against the distal portion of the endoscope during treatment. It is envisioned that the suction provided by the vacuum may be pulsed or otherwise interrupted to permit fragments or dust generated by the fracturing of the kidney or bladder stone to be removed from the surgical site. It is contemplated that a distal portion of the first channel may include a protrusion, such as an annular boss or protuberance, that causes the fragments or dust to be captured thereon or freely flow through the first channel. As can be appreciated, the duration of the vacuum pulse is such that the kidney or bladder stone being treated is not move outside the suction capability of the vacuum source.

The distal portion of the endoscope includes a second aperture defined therein that is in fluid communication with a second channel defined within the elongate body of the endoscope. The second channel is in fluid communication with an irrigation source such that irrigation fluid may be transported to the treatment site. It is envisioned that the second aperture may be disposed on the endoscope at a location other than the distal portion, such as an outer surface thereof.

The distal portion of the endoscope includes a third aperture defined therein that is in mechanical communication with a third channel defined within the elongate body of the endoscope. The second channel includes an optical waveguide or other suitable electromagnetic wave transmission device disposed therein to transmit energy generated by a laser that is in electromagnetic communication therewith. It is contemplated that the laser may be disposed within a portion of the endoscope or disposed remote from the endoscope. In embodiments, the optical waveguide is rotatably supported within the second channel and includes a beveled tip that causes the energy generated by the laser to propagate at an angle relative to a longitudinal axis defined by the elongate body of the endoscope. As such, rotation of the optical waveguide causes the beveled tip thereof to be oriented in various directions, thereby altering the direction in which energy generated by the laser propagates therefrom. It is envisioned that the optical waveguide may be fixedly disposed within the second channel and the distal portion of the endoscope may include a lens disposed thereon having a beveled edge. In this embodiment, rotation of the lens alters the direction in which the energy generated by the laser propagates therefrom. In embodiments, rotation of the optical waveguide or lens, and actuation of the laser, may be performed manually by the clinician or automatically by the computing device using the image data captured by the ultrasound sensor.

In embodiments, the endoscope may include a stationary portion and an extendible portion in mechanical communication therewith. In this manner, the endoscope may be navigated adjacent, but spaced apart from, the kidney or bladder stone intended to be treated. The stationary portion of the endoscope is immobilized using a balloon or other suitable mechanism and the extendible portion is extended either manually or automatically until the distal portion of the endoscope is in contact with the kidney or bladder stone intended to be treated.

It is envisioned that the endoscope may be a rigid endoscope or a flexible endoscope. In the case where the endoscope is rigid, it is contemplated that the endoscope may include a manipulatable distal segment that may be manipulated relative to the longitudinal axis defined by the endoscope. The manipulatable distal segment may include an internally reflected laser steering system to alter the direction in which energy generated by the laser propagates from the distal portion of the endoscope.

An endoscope for treating benign prostate hyperplasia (BPH) is also provided and includes an ultrasonic sensor similar to that described herein above. The BPH endoscope also includes an aperture defined within a distal portion thereof that is in mechanical communication with a channel defined through an elongate body of the BPH endoscope. A shaver is slidably and rotatably disposed within the channel and includes a distal tip capable of dissecting and removing portions of a transitional zone capsule being treated. In this manner, the distal tip of the shaver may be a rotary or reciprocal shaver and includes one or more sharpened edges capable of cutting tissue. A throughbore is defined through the shaver adjacent the sharpened edges that is in fluid communication with the vacuum source such that tissue dissected by the distal tip of the shaver is removed from the surgical site. As can be appreciated, use of the ultrasound sensor enables the clinician to more easily visualize and identify the patient's bladder neck, thereby enabling the clinician to better preserve the bladder neck and improve retrograde ejaculation outcomes. Further, use of the ultrasound sensor enables the clinician to more easily monitor the depth of the transitional tissue in real time and enables the clinician to continue treatment of the transitional zone capsule without the need to pause for hemostasis, thereby enabling the clinician to complete treatment of the transitional zone capsule more rapidly and permits a more complete dissection thereof.

Embodiments of the disclosure are now described in detail with reference to the drawings in which like reference numerals designate identical or corresponding elements in each of the several views. In the drawings and in the description that follows, terms such as front, rear, upper, lower, top, bottom, and similar directional terms are used simply for convenience of description and are not intended to limit the disclosure. As used herein, the term "clinician" refers to a doctor, a nurse, or any other care provider and may include support personnel. Throughout this description, the term "proximal" refers to the portion of the device or component thereof that is closer to the clinician and the term "distal" refers to the portion of the device or component thereof that is farther from the clinician. In the following description, well-known functions or constructions are not described in detail to avoid obscuring the disclosure in unnecessary detail.

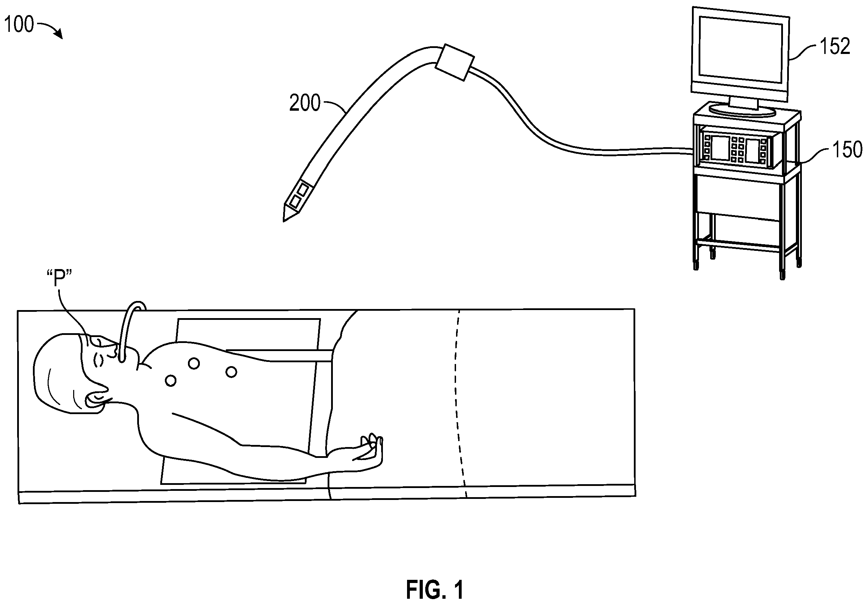

Referring now to the drawings, a system for treating kidney and bladder stones is provided and generally identified by reference numeral 100. As illustrated in FIGS. 1-8, the system 100 includes a computing device 150 and an endoscope 200 for navigating to and treating a kidney stone of a patient "P." Although generally described as being utilized to treat a kidney stone, it is contemplated that the system 100 may be utilized to treat any similar ailment, such as bladder stones or the like. The computing device includes a processor (not shown) and a memory (not shown) in electrical communication therewith. The memory is configured to store a software application, which when executed, is configured to process images captured by the endoscope 200 using radiomics or other similar means, as will be described in further detail hereinbelow. The endoscope 200 is coupled to the computing device 150 and it is contemplated that either the endoscope 200 or the computing device 150 may include the memory and/or processor disposed therein.

The endoscope 200 includes an elongate body configured to be advanced within a lumen of a patient, such as the urethra. As can be appreciated, during treatment of kidney stones the endoscope 200 may be a ureteroscope, and during treatment of bladder stones the endoscope 200 may be a cystoscope. In this manner, during treatment of kidney stones, the endoscope 200 may include one or more flexible segments capable of enabling the endoscope 200 to be manipulated relative to a longitudinal axis defined by the endoscope 200 and therefore, more easily navigate the bladder of the patient, enter a ureter, and navigate the ureter to reach one or more kidney stones located therein. In contrast, during the treatment of bladder stones, the endoscope 200 may be rigid.

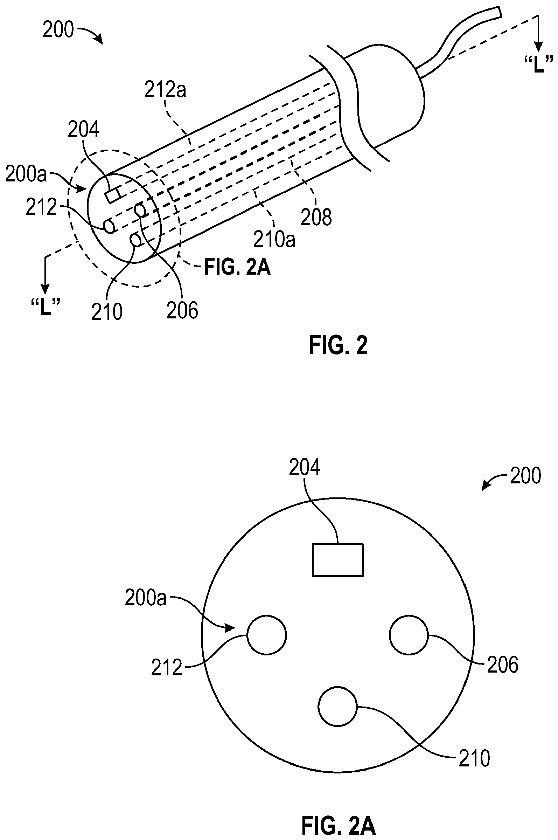

FIG. 2A illustrates a distal portion 200a of the endoscope 200, which includes an ultrasound sensor 204 disposed thereon for generating a two-dimensional (2D), or in embodiments, a three-dimensional (3D) representation of the patient's anatomy disposed distal of the endoscope 200. The ultrasound sensor 204 may be a miniaturized ultrasound sensor, such as a capacitive micromachined ultrasound transducer (CMUT) or a piezoelectric micromachined ultrasonic transducer (PMUT). As can be appreciated, the use of the ultrasound sensor 204 during cystoscopy or ureteroscopy (*e.g.,* procedures in which the endoscope 200 is submerged in a fluid) provides a clearer image of the patient's anatomy distal of the endoscope 200, and therefore, enables a clinician to more easily navigate the patient's luminal network to reach the kidney or bladder stone in addition to more accurately visualizing the kidney or bladder stone during treatment. In contrast with traditional optical endoscopes where a user must inspect each segment of the luminal network for stones, the ultrasound guided endoscope can see though the luminal walls to find kidney stones that would otherwise be hidden from direct view. Once a stone identified the, ultrasound sensor 204 enables the clinician to navigate to and place the distal portion 200a of the endoscope 200 adjacent the kidney or bladder stone rather than maintaining a gap between the distal portion 200a of the endoscope 200 and the kidney and bladder stone to maintain a visual of the kidney or bladder stone, as is the case with an endoscope 200 including an optical camera or the like.

The ultrasound sensor 204 is in electrical communication with the computing device 150 such that the reflected ultrasound signals received by the ultrasound sensor 204 are processed by the processor of the computing device 150 using radiomics. In this manner, the processor of the computing device 150 processes the image data captured by the ultrasound sensor 204 and generates a 2D ultrasound image, or in embodiments, a 3D image of the patient's anatomy and displays the generated image on a display screen 152 associated with the computing device 150 (FIG. 1). In embodiments, it is contemplated that the endoscope 200 may include the processor (not shown) or other suitable computing device capable of generating the 2D or 3D image and transmitting the 2D or 3D image data to the computing device 150 to be displayed on the display 152.

The computing device 150 may register the images of the patient anatomy to fiducials on the patient or to images of the patient anatomy derived preoperatively from CT or MRI images.

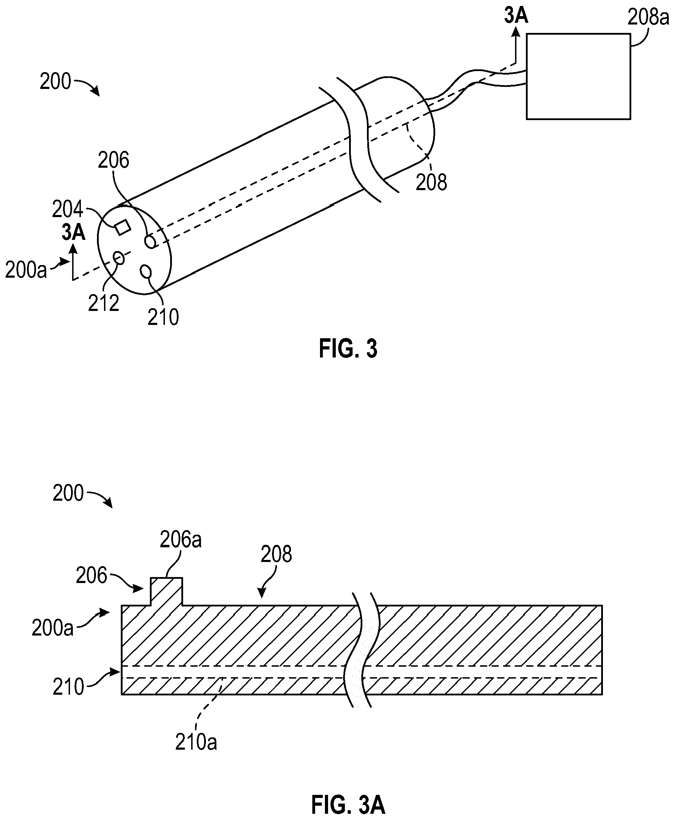

The distal portion 200a of the endoscope 200 includes a first aperture 206 defined therein and in fluid communication with a first channel 208 defined within the elongate body of the endoscope 200 (FIG. 3). The first channel 208 is in fluid communication with a fluid source (not shown) for delivering a fluid to the distal portion 200a of the endoscope 200 and vacuum source 208a (FIG. 3) disposed remote from the endoscope 200. The vacuum source 208a is configured to apply a vacuum to the distal portion 200a of the endoscope 200 to cause the kidney or bladder stone being treated to be held against the distal portion 200a of the endoscope 200 for treatment, as will be described in further detail hereinbelow.

The vacuum source 208a is in electrical communication with a control system (not shown) associated with the endoscope 200. The control system may be disposed on a portion of the endoscope 200 for manipulation by the clinician, or in embodiments, may be disposed remote from the endoscope 200. The vacuum source 208a may be activated and inactivated by manipulation of the control system by the clinician. It is contemplated that when the vacuum source 208a is activated, the vacuum source 208a may pulse the application of a vacuum at the first aperture 206 to allow stone fragments or stone dust that may be occluding the first aperture 206 to be cleared. As can be appreciated, the duration of the vacuum pulsing by the vacuum source 208a may be of sufficiently short duration to permit the occluding stone fragments or stone dust to be cleared, while at the same time ensuring that the stone being treated remains in contact with the distal portion 200a thereby ensuring that the stone does not move outside of the suction capability of the vacuum source 208a.

As illustrated in FIG. 3A, a portion of the first channel 208 adjacent the first aperture 206 defines a protrusion 206a thereon that causes stone fragments and stone dust to either collect on the protrusion 206a or flow freely into the first aperture 206 and through the first channel 208. It is contemplated that the protrusion 206a may be in the form of an annular boss (*e.g.,* reduction in inner diameter of the first channel 208), one or more protuberances, etc. such that the cross-sectional area of the first channel 208 is reduced adjacent the first aperture 206.

The distal portion 200a of the endoscope 200 includes a second aperture 210 defined therein that is in fluid communication with a second channel 210a defined within the elongate body of the endoscope 200 (FIG. 2). The second channel 210a is in fluid communication with an irrigation source (not shown) such that irrigation fluid may be transported to the treatment site. Although generally described as being disposed on the distal portion 200a of the endoscope 200, it is contemplated that the second aperture 210 may be defined on any suitable portion of the endoscope 200 capable of delivering irrigation fluid to the treatment site, such as on an outer surface of the endoscope 200 (*e.g.,* an outer diameter thereof), etc. The second channel 210 may additionally or alternatively allow for passage of an optical waveguide, as described elsewhere herein, along with the irrigation fluid. The irrigation fluid serving to maintain the surgical field (e.g., enabling ultrasound transmission to image the tissue) as well as clearing the lumen and controlling the temperature of the optical waveguide.

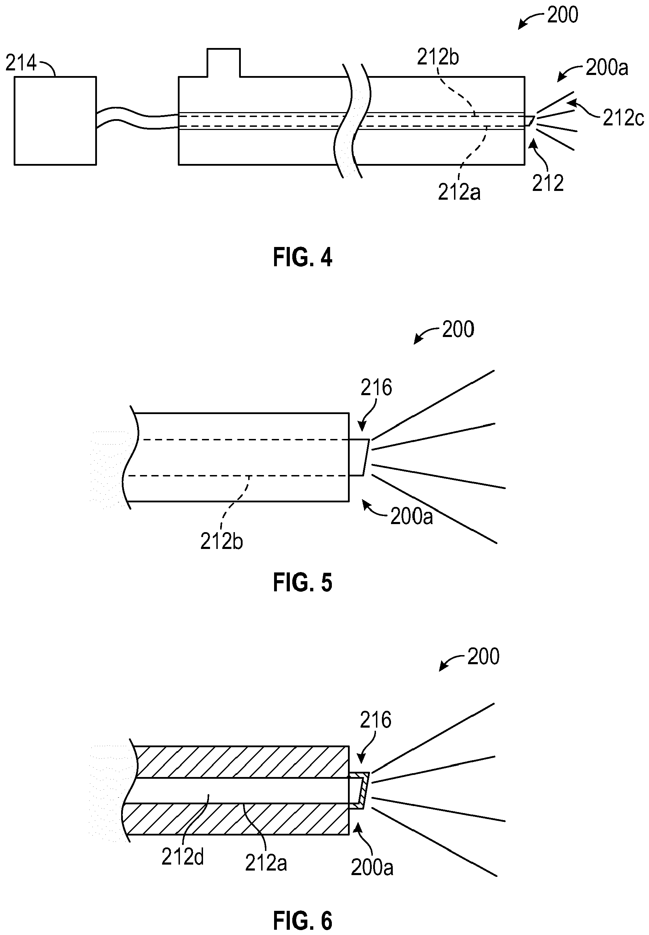

The distal portion 200a of the endoscope 200 includes a third aperture 212 defined therein that is in mechanical communication with a third channel 212a defined within the elongate body of the endoscope 200 (FIGS. 2 and 4). The second channel 212a includes an optical waveguide 212b or other suitable electromagnetic wave transmission device such as optical fibers, light pipes, etc. in one non-limiting embodiment, the optical waveguide 212b is an optical fiber configured to transmit energy generated by a laser 214 in electromagnetic communication therewith. The laser 214 may be disposed within a portion of the endoscope 200 or may be disposed remote therefrom and may be selectively controlled by the clinician by means of the control system (not shown). A distal portion of the optical waveguide 212b may include a tapered or otherwise beveled tip 212c (FIG. 4) capable of directing propagation of the energy generated by the laser 214 at an angle that is incident to the longitudinal axis of the endoscope 200. In this manner, the optical waveguide 212b is rotatably supported within the third channel 212a and is in mechanical communication with a toggle (not shown) or other suitable device capable of rotating the optical waveguide 212b within the third channel 212a to manipulate the direction of propagation of the energy generated by the laser 214. In embodiments, the toggle may be a device, such as a slide or other similar mechanism that is manipulatable by the clinician to cause manual rotation of the optical waveguide 212b within the third channel 212a. In certain embodiments, the toggle may be a button or other device that is in electrical communication with a motor or the like, such that manipulation of the button by the clinician causes the motor to effectuate rotation of the optical waveguide 212b within the third channel 212a.

As can be appreciated, the direction in which the energy generated by the laser 214 propagates causes different portions of the kidney or bladder stone being treated to break apart. In this manner, the clinician may manipulate the direction in which the energy generated by the laser 214 propagates and therefore impacts the kidney or bladder stone. Continued manipulation of the energy generated by the laser 214, in combination with the images generated by the ultrasound sensor 204, enables the clinician to ensure that the entirety of the kidney or bladder stone being treated is removed from the patient. In this manner, the computing device 150 utilizes radiomic segmentation of the kidney or bladder stone being treated to accurately depict the status of the kidney or bladder stone on the display screen 152 of the computing device 150. It is contemplated that the computing device 150 may display the target of the laser energy on the depiction of the kidney or bladder stone being treated, such that the clinician may visualize where the energy generated by the laser 214 is being focused.

In embodiments, it is contemplated that the optical waveguide 212b may be fixedly disposed within the third channel 212a or an inner surface 212d (FIG. 6) of the third channel 212a may act as the optical waveguide 212b using any suitable means, such as coating the inner surface 212d with a suitable coating capable of reflecting the laser energy along a length of the third channel 212a. A lens 216 (FIGS. 5 and 6) is rotatably disposed over the third aperture 212 on the distal portion 200a of the endoscope 200 such that the lens 216 is in electromagnetic communication with the optical waveguide 212b. The lens 216 defines a beveled or otherwise angled surface 216a capable of directing propagation of the energy generated by the laser 214 at an angle that is incident to the longitudinal axis of the endoscope 200. It is contemplated that the lens 216 may be formed from a solid material, may be a fluidic lens, may be a micro-electromechanical system (MEMS) mirror, etc. Further, the lens may be replaced by a mirror or mirror set within a limited distance of the distal end of the optical waveguide.

To alter the direction in which the energy generated by the laser 214 propagates, the lens 216 is selectively manipulatable by the clinician using the control system (not shown) to rotate the lens 216 about a longitudinal axis defined by the third channel 212a. It is contemplated that manipulation of the lens 216 by the clinician may be effectuated using similar means to manipulation of the rotatable optical waveguide 212b described hereinabove.

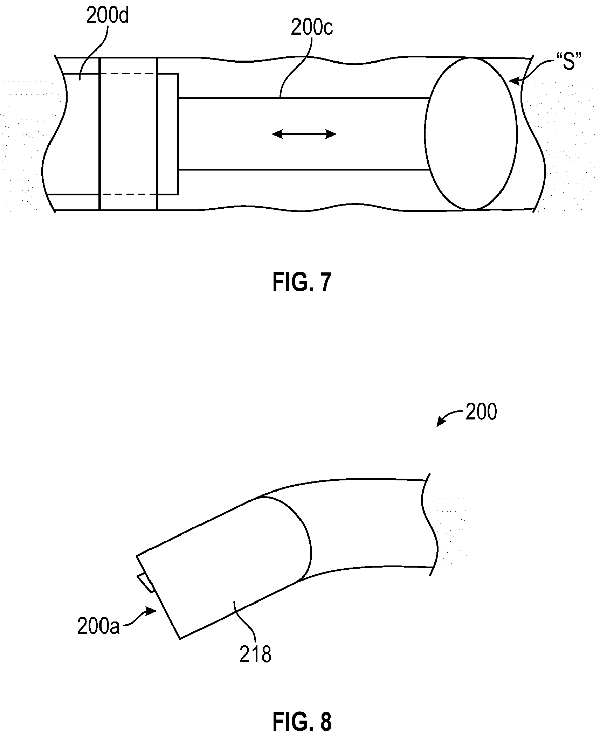

It is envisioned that the direction in which energy generated by the laser 214 propagates and the generation of energy by the laser 214 may be automatically controlled by the computing device 150. In this manner, the computing device 150 utilizes the data captured by the ultrasonic sensor 204 to identify portions of the kidney or bladder stone to be exposed to the energy generated by the laser 214. As the kidney or bladder stone is fragmented, the ultrasound sensor 204 continues to transmit updated image data to the computing device 150 such that the 2D or 3D representation of the kidney or bladder stone being treated is continuously updated. As such, the computing device 150 continuously updates the direction in which the energy generated by the laser 214 should propagate, and automatically controls the orientation of the optical waveguide 212b or lens 216 to direct the propagation of the energy generated by the laser 214 thereon until the kidney or bladder stone being treated is completely removed from the patient. In embodiments, as the clinician navigates the endoscope 200 to the kidney or bladder stone, the clinician may anchor or otherwise ensure the location of the endoscope 200 is maintained at a distance from the kidney or bladder stone "S" using any suitable means, such as a balloon, selectively activatable protrusions, etc. (FIG. 7). The endoscope 200 may include an extendible portion 200c that is in mechanical communication with a stationary portion 200d of the endoscope. The computing device 150, utilizing image data obtained by the ultrasound sensor 204, automatically controls the extension of the extendible portion 200c of the endoscope 200 to the kidney or bladder stone "S" to be treated. It is envisioned that the computing device 150 is in electrical communication with an extension motor (not shown) that is in mechanical communication with the extendible portion 200c of the endoscope 200 to effectuate extension and retraction thereof. It is contemplated that the extension motor may be disposed at a mid-portion of the endoscope 200 or may be disposed at a proximal portion thereof.

To extend the distal portion 200a of the endoscope 200 to the kidney or bladder stone "S" the computing device 150 automatically causes the extension motor to effectuate extension of the extendible portion 200c of the endoscope 200. The computing device 150 utilizes the continuously updated image data obtained by the ultrasound sensor 204 to monitor the location of the distal portion 200a of the endoscope 200 relative to the kidney or bladder stone "S" and automatically controls extension of the extendible portion 200c in accordance therewith. In this manner, the computing device 150 ensures that the first aperture 206 is in close enough proximity to the kidney or bladder stone "S" to ensure that the suction generated by the vacuum source 208a causes the kidney or bladder stone "S" to maintain its position relative to the distal portion 200a of the endoscope.

As described herein above, it is contemplated that the endoscope 200 may be a flexible endoscope or may be a rigid endoscope, depending upon the surgical procedure being performed. In embodiments, it is contemplated that a rigid endoscope 200 may include at least one distal manipulatable segment 218 (FIG. 8), such that a distal portion of the endoscope 200 may be manipulated relative to the longitudinal axis defined by the endoscope 200. In this manner, the clinician may selectively manipulate the distal manipulatable segment 218 using the control system (not shown) or manually using a toggle or the like (not shown). It is contemplated that the distal manipulatable segment 218 may include an internally reflected laser steering system (not shown) capable of directing propagation of the energy generated by the laser 214 in any suitable direction. As can be appreciated, the reflected laser steering system may be selectively controlled by the clinician or automatically controlled by the computing device 150 utilizing the image data captured by the ultrasound sensor 204.

In operation, the endoscope 200 is initially advanced within a lumen of the patient, such as the urethra. The ultrasound sensor 204 captures image data of the patient's anatomy and the processor of the computing device 150 processes the captures data and generates a representation of the patient's anatomy and displays the generated representation of the patient's anatomy on the display screen 152. At this point, the endoscope 200 is further advanced within the lumen of the patient and is navigated to the kidney stone to be treated using the representation of the patient's anatomy that is displayed on the display 152. Once the distal portion 200a of the endoscope is disposed adjacent the kidney stone to be treated, the vacuum source 208a is activated to cause suction to be generated at the first aperture 206 of the first channel 208. The suction generated at the first aperture 206 causes the kidney stone to be treated to held against the distal portion 200a of the endoscope 200. The image data of the kidney stone captured by the ultrasound sensor 204 is processed by the processor of the computing device 150 and a representation of the kidney stone is displayed on the display screen 152. At this point, energy is generated by the laser 214 and is propagated through the optical waveguide 212b. The laser energy continues to propagate through the optical waveguide 212b and exits through the beveled tip 212c of the optical waveguide, and thereafter, is applied to the kidney stone to cause the kidney stone to begin to fragment. The overall size and shape of the kidney stone is monitored on the display screen as the representation of the kidney stone is continuously updated by the processor of the computing device 150. Using the representation of the kidney stone displayed on the display screen 152, the direction in which the laser energy propagates, and therefore, the location at which the laser energy is focused on the kidney stone is manipulated by either manually or automatically rotating the optical waveguide 212b to alter the direction in which the beveled tip 212c of the optical waveguide 212b is oriented. As can be appreciated, in the case where the optical waveguide 212b is fixedly disposed within the endoscope 200, the lens 216 may rotated either manually or automatically such that direction in which the beveled surface 216a of the lens is oriented may be adjusted similarly to that described herein above with respect to the rotatable optical waveguide. During the application of laser energy to the kidney stone, the vacuum generated by the vacuum source 208a may be pulsed such that fragments and dust created by the fragmentation of the kidney stone by the laser energy may enter the first aperture 206 and be removed from the surgical site.

Using the representation of the kidney stone displayed on the display screen 152, total removal of the kidney stone being treated can be monitored and identified. Once total removal of the kidney stone being treated is accomplished, the endoscope 200 is removed from the patient or may be navigated to another kidney stone should it be necessary. The above steps may be repeated as many times as necessary to treat any number of kidney stones.

In accordance with embodiments of the disclosure, the endoscope may include a multi axis accelerometer sensor. Such an accelerometer provides pitch and roll information of the orientation of a distal portion of the endoscope. Pitch and roll information can be useful in depicting the orientation of ultrasound image to the patient anatomy. Additionally, or alternatively, the endoscope may include a navigation sensor. The navigation sensor may be a shape sensor defining the shape of a distal portion of the endoscope. This shape can be matched to luminal shapes of various portions of the patient's anatomy to determine where in the anatomy the endoscope is located. Additionally or alternatively, the sensor may be an electromagnetic (EM) sensor. If an EM sensor is employed, the patient may be placed on an electromagnetic field generator and the position of the EM sensor within that field detected during the procedure. One example of an EM filed generator is the Aurora EM tracking system offered by Norther Digital Inc. Using the EM field, the patient can be registered to pre-procedure images such as those from CT and MRI systems. This registration can provide guidance to the clinician for navigation of the endoscope through the anatomy of the patient.

Although generally described as being manually controlled, in the case where the endoscope 200 includes an extendible portion 200c, the endoscope 200 is navigated to a location that adjacent to but spaced apart from the kidney stone to be treated. Using the representation of the patient's anatomy generated by the computing device 150, the computing device 150 causes the stationary portion 200d of the endoscope to be anchored or otherwise immobilized relative to the lumen of the patient. At this point, the computing device 150 causes the extendible portion 200c of the endoscope to extend towards the kidney stone to be treated. Once adjacent to the kidney stone to be treated, the computing device 150 causes the vacuum source 208a to generate suction at the first aperture 206 and cause the kidney stone to be treated to held against the distal portion 200a of the endoscope. Treatment of the kidney stone using the laser 214 is similar to that described hereinabove and therefore will not be described in detail herein in the interest of brevity.

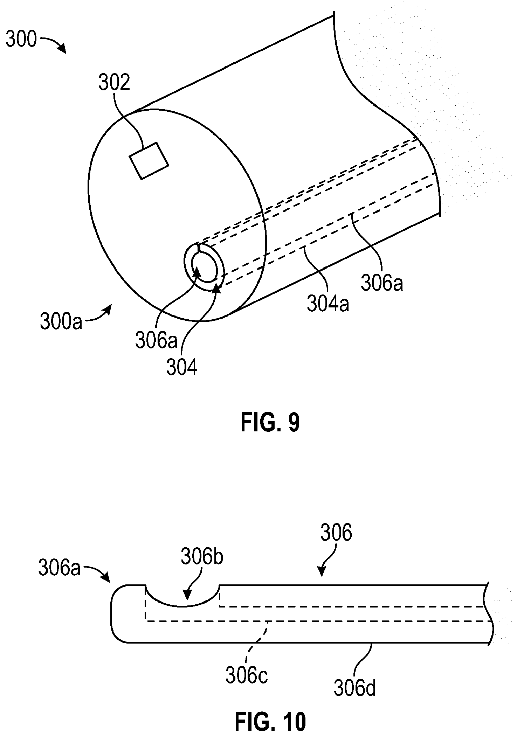

Referring to FIGS. 9 and 10, an endoscope for navigating to and treating benign prostate hyperplasia is provided and generally identified by reference numeral 300. The endoscope 300 is coupled to the computing device 150 and either the endoscope 300 or the computing device 150 is configured to process images captured by the endoscope 300 using radiomics or other similar means similarly to endoscope 200 described hereinabove.

In this manner, a distal portion 300a of the endoscope 300, which includes an ultrasound sensor 302 disposed thereon for generating a two-dimensional (2D) or three-dimensional (3D) image of the patient's anatomy located distal of the endoscope 300. The ultrasound sensor 302 is in electrical communication with the computing device 150 and may be a miniaturized ultrasound sensor, such as a capacitive micromachined ultrasound transducer (CMUT) or a piezoelectric micromachined ultrasonic transducer (PMUT). As can be appreciated, the use of the ultrasound sensor 302 during transurethral resection of the prostate (TURP) or transurethral holmium laser enucleation of the prostate (HoLEP) provides a clearer image of the patient's anatomy distal of the endoscope 300 during resection, and in particular, as the transitional zone capsule bleeds, which would ordinarily obstruct the clinicians view of the transitional zone capsule when using an optical scope. Specifically, displaying the image data captured by the ultrasound sensor 302 on the display screen 152 associated with the computing device 150 enables the clinician to more easily monitor the depth of the transitional tissue in real-time and enables the clinician to continue treatment of the transitional zone capsule without the need to pause for hemostasis. thereby enabling the clinician to complete treatment of the transitional zone capsule more rapidly and permits a more complete dissection thereof. Additionally, it is envisioned that the use of the ultrasound sensor 302 enables the clinician to more easily visualize and identify the patient's bladder neck, thereby enabling the clinician to better preserve the bladder neck and improve retrograde ejaculation outcomes.

The distal portion 300a of the endoscope 300 includes a first aperture 304 defined therein which is in mechanical communication with a first channel 304a within the elongate body of the endoscope 300. A shaver 306 is disposed within the first channel 304a and is configured to selectively extend through the first aperture 304. The shaver 306 may be any suitable shave known in the art and in embodiments may be a rotary or reciprocal shaver having a distal tip 306a capable of dissecting and removing portions of the transitional zone capsule being treated. In this manner, the distal tip 306a may include one or more sharpened edges 306b defined thereon adjacent a throughbore 306c defined through an elongate body 306d of the shaver 306. In embodiments, the throughbore 306c of the shaver 306 may be in fluid communication with the vacuum source 208a such that the portions of the transitional zone capsule removed by the shaver 306 are removed from the surgical site. As can be appreciated, the use of the shaver 306 enables the clinician to quickly remove tissue and/or morcellate the tissue thereby reducing treatment time and improving surgical outcomes.

Operation of the endoscope 300 is similar to that of the endoscope 200 described hereinabove, and therefore, only the differences therebetween will be described herein in the interest of brevity.

Once the distal portion 300a of the endoscope 300 is disposed adjacent to the transitional zone capsule to be treated, the distal tip 306a of the shaver 306 is extended towards the transitional zone capsule and begins to dissect the tissue therein. As can be appreciated, as the tissue is dissected, the tissue begins to bleed and ordinarily visibility will begin to be impeded. However, the ultrasound sensor 302 is utilized to generate a representation of the transitional zone capsule as it is being dissected, such that the overall size of the transitional zone capsule may be monitored on the display screen 152 and efficient removal of the transitional zone capsule being treated may be accomplished. The representation of the transitional zone capsule is continuously updated using the image data captured by the ultrasound sensor 302 and an accurate representation is able to be generated regardless of the presence of blood or other fluids between the distal portion 300a of the endoscope and the transitional zone capsule being treated.

As the shaver 306 dissects and/or morcellate the transitional zone tissue, the vacuum source 208a causes the dissected/morcellated tissue to be removed from the surgical site. Using the representation of the transitional zone capsule displayed on the display screen 152, total removal of the transitional zone tissue can be monitored and identified. Once total removal of the transitional zone tissue is accomplished, the endoscope 300 is removed from the patient or may be navigated to another location to additional treatment. The above steps may be repeated as many times as necessary to treat any number of locations within the patient.

Surgical instruments such as the endoscopes, computing devices, and other components of systems 100 described herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, endoscopes, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

It is contemplated that the endoscopes described herein may be positioned by the robotic system and the precise position of the endoscope transmitted to the computer to construct the 3D image of the ultrasonic imaged organ, stone, or operative field. The robotic system has the ability to autonomously scan the surgical field and construct a complete 3D model of the field to aid the surgeon in directing the robotic arms or to provide necessary 3D information for the robotic system to further conduct surgical steps autonomously. The robotic system provides the relative coordinates between respective endoscopes needed to triangulate the points in the live ultrasound images and views to construct a 3D surface of the operative field.

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

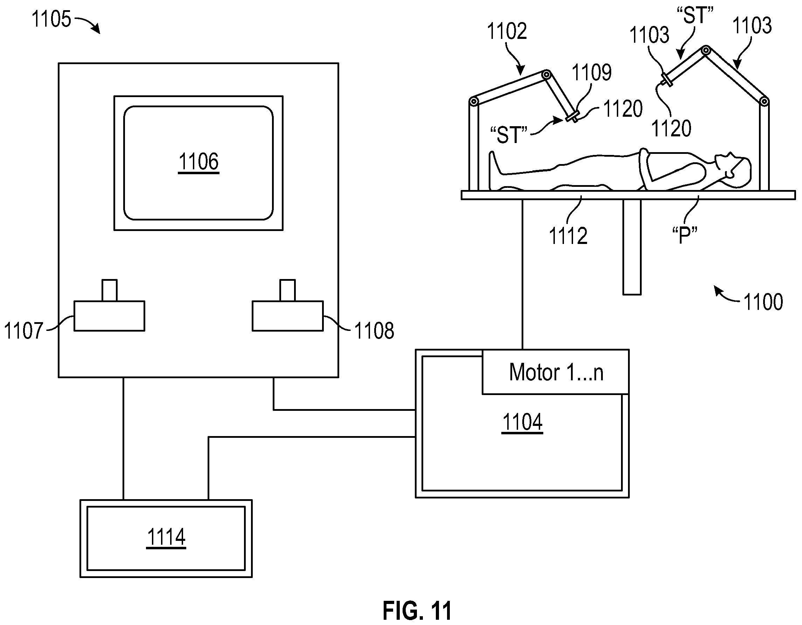

Referring to FIG. 11, a medical workstation is shown generally as workstation 1100 and generally may include a plurality of robot arms 1102, 1103, a control device 1104, and an operating console 1105 coupled with control device 1104. Operating console 1105 may include a display device 1106, which may be set up in particular to display 3D images, and manual input devices 1107, 1108, by means of which a person (not shown), for example a surgeon, may be able to telemanipulate robot arms 1102, 1103 in a first operating mode.

Each of the robot arms 1102, 1103 may include a plurality of members, which are connected through joints, and an attaching device 1109, 1111, to which may be attached, for example, a surgical tool "ST" supporting an end effector 1120, in accordance with any one of several embodiments disclosed herein, as will be described in greater detail below. For example, the end effector 1120 may be an ultrasound endoscope as described herein above.

Robot arms 1102, 1103 may be driven by electric drives (not shown) that are connected to control device 1104. Control device 1104 (e.g., a computer) may be set up to activate the drives, in particular by means of a computer program, in such a way that robot arms 1102, 1103, their attaching devices 1109, 1111 and thus the surgical tool (including end effector 1120) execute a desired movement according to a movement defined by means of manual input devices 1107, 1108. Control device 1104 may also be set up in such a way that it regulates the movement of robot arms 1102, 1103 and/or of the drives.

Medical workstation 1100 may be configured for use on a patient "P" lying on a patient table 1112 to be treated in a minimally invasive manner by means of end effector 1120. Medical workstation 1100 may also include more than two robot arms 1102, 1103, the additional robot arms likewise being connected to control device 1104 and being telemanipulatable by means of operating console 1105. A medical instrument or surgical tool (including an end effector 1120) may also be attached to the additional robot arm. Medical workstation 1100 may include a database 1114, in particular coupled to with control device 1104, in which are stored, for example, pre-operative data from patient/living being "P" and/or anatomical atlases.

A 3D model of the patient anatomy may be presented to the surgeon in a way such that the surgeon may define portions of the anatomy intended for resection or ablation. Once the region is defined, the robot may begin automatic resection of the identified tissue and with the advantage of monitoring resection through ultrasound image. The robot may track natural fiducials with the original image in order to update the 3D anatomy and the originally identified tissue for resection. Should the anatomy be distorted beyond the a establish safety profile, a new 3D model of the remaining anatomy may be presented to the surgeon in a repeat of the identification and resection process.

Yet a further aspect of the disclosure relates to the use of an ultrasound guided endoscope as described herein, in connection with sensors such as Fiber-Bragg gratings or other shape sensors to detect the shape of the endoscope. As outlined below, such an arrangement has particular benefits for luminal navigation in the context of lung navigation.

There exist a number of systems that utilize the output from a pre-procedural computed tomography (CT) scan (e.g., CT image data) for purposes of identifying areas of interest or targets to which navigation of an endoscope or catheter is desired. Typically, this navigation will be of luminal networks such as the airways of the lungs or the biliary tract, but they could also be of spaces such as the thoracic cavity generally or other locations within a patient. These systems generally have two phases. A first phase is a planning phase where the targets are identified and a three-dimensional (3D) model is generated. A second phase is a navigation phase where the location of the catheter within the patient is detected and depicted on the 3D model or other images to allow the clinician to navigate to the identified targets. By updating the position of a catheter within the 3D model, the clinician can perform procedures such as biopsy or treatment at the target location.

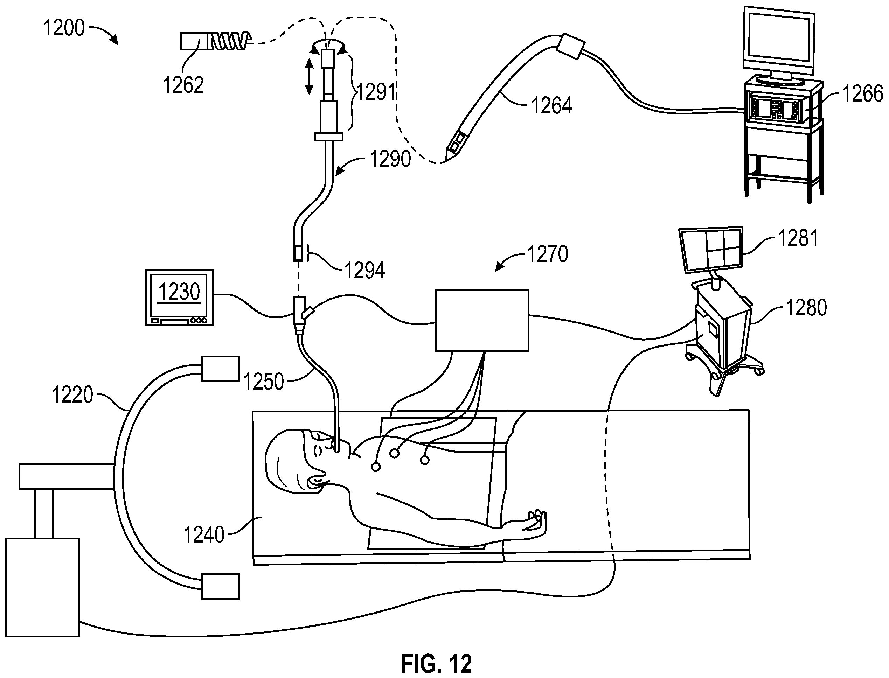

FIG. 12 depicts a system 1200 suitable for implementing methods described herein. As shown in FIG. 1, system 1200 is used to perform one or more procedures on a patient supported on an operating table 1240. In this regard, system 1200 generally includes an optical bronchoscope 1250, monitoring equipment 1230, a tracking system 1270, and a computing device 1280.

Bronchoscope 1250 is configured for insertion through the patient's mouth and/or nose into the patient's airways. Bronchoscope 1250 includes a source of illumination and a video imaging system (not explicitly shown) and is coupled to monitoring equipment 1230, for example, a video display, for displaying the video images received from the video imaging system of bronchoscope 1250. In one embodiment of the disclosure the optical bronchoscope 1250 may operate in combination with an ultrasound bronchoscope 1290. The ultrasound bronchoscope 1290 may be configured as described elsewhere herein. The ultrasound bronchoscope 1290 may act as an extended working channel (EWC) of the optical bronchoscope and be configured for insertion through a working channel of optical bronchoscope 1250 into the patient's airways (although the ultrasound bronchoscope 1290 may alternatively be used without optical bronchoscope 1250). Ultrasound bronchoscope 1290 includes a handle 1291 which can be manipulated by rotation and compression to steer ultrasound bronchoscope 1290. In one embodiment the ultrasound bronchoscope 1290 includes a shape sensor 1294. The shape sensor may be for example a Fiber-Bragg grating sensor or another sensor associated with the ultrasound bronchoscope 1290 from which the shape of a distal portion of the ultrasound bronchoscope 1290, and an orientation of distal face of the ultrasound bronchoscope (e.g., ultrasound sensor 204).

Data output by the shape sensor 1294 is received by a tracking system 1270. One aspect of the tracking system 1270 is to determine the shape of the ultrasound bronchoscope 1290 an match the shape to shapes found in a 3D model of the lungs generated from pre-procedure CT images (or other imaging modalities) By comparing the shape of the ultrasound bronchoscope 1290, or a portion of the ultrasound bronchoscope 1290, the tracking system 1270 can determine where in the 3D model and thus where in the patient's lungs the ultrasound bronchoscope 1290 has been navigated.

According to an embodiment, biopsy and treatment tools 1262, 1264 are configured to be insertable into ultrasound bronchoscope 1290 following navigation to a target location. Biopsy tool 1262 may be used to collect one or more tissue samples from the target location In one example, treatment tool 1264 is configured to be operated with a generator 1266, such as a radio frequency generator or a microwave generator and may include any of a variety of ablation tools and/or catheters. Though shown as a biopsy tool and microwave ablation tool in FIG. 12, those of skill in the art will recognize that other tools including for example RF ablation tools, brachytherapy tools, and others may be similarly deployed and tracked without departing from the scope of the present disclosure.

Utilization of the system 1200 during a procedure will now be described. Using computing device 1280, various views of the image data and/or a 3D model may be displayed to and manipulated by a clinician to facilitate identification of the target location. Th image data and 3D models may be derived from pre-procedure CT, MRI, PET, fluoroscopy and other images. A target location is a site within the patient's lungs where treatment or biopsy is to be performed. For example, the treatment location may be located in lung tissue adjacent to an airway. The 3D model may include, among other things, a model airway tree corresponding to the actual airways of the patient's lungs, and show the various passages, branches, and bifurcations of the patient's actual airway tree. Additionally, the 3D model may include lesions, markers, blood vessels and vascular structures, lymphatic vessels and structures, organs, other physiological structures, and/or a 3D rendering of the pleural surfaces and fissures of the patient's lungs. Some or all of the aforementioned elements may be selectively displayed, such that the clinician may choose which elements should be displayed when viewing the 3D model.

After identifying the target location, application 1281 may determine a pathway between the patient's trachea and the target location via the patient's airways. In instances where the target location is located in lung tissue that is not directly adjacent an airway, at least a portion of the pathway will be located outside of the patient's airways to connect an exit point on an airway wall to the target location.

With the pathway determined for navigation of the ultrasound bronchoscope 1290, as an initial step of the procedure a registration is undertake. When using a 3D model generated from CT scan (or other imaging modality), the 3D model must be registered with the patient's actual airways to enable application 1281 to display an indication of the location of the ultrasound bronchoscope 1290, using the shape sensor 1294, in the 3D model. The registration is necessary because the CT scan may have been taken days, and even weeks or months prior to the actual procedure. Even if the CT scan were taken the same day, such CT scans are not undertaken within a surgical suite thus registration is still necessary.

One potential method of registration involves performing a survey of the patient's lungs by the ultrasound bronchoscope 1290 with shape sensor 1294 into each lobe of the patient's lungs to at least the second bifurcation of the airways of that lobe. The shape of the ultrasound bronchoscope 1290 as it is navigated is tracked during this registration phase, and a model is generated representing the shape of the airways navigated by the ultrasound bronchoscope 1290 during the sweep is generated. The model from the sweep is matched to 3D model to finalize the registration. While the registration process focuses on aligning the patient's actual airways with the airways of the 3D model, registration also ensures that the position of vascular structures, pleural surfaces, and fissures of the lungs are accurately determined.

Once the pre-procedure images and 3D model are registered to the patient's lungs, the optical bronchoscope 1250 may be navigated along the pathway until it becomes wedged in the airways. From that point forward, the ultrasound bronchoscope 1290 is navigated along the pathway until proximate the target location. Once proximate the target, an access tool, such as a piercing or puncture tool, inserted into the ultrasound bronchoscope 1290 to create an opening in the airway wall. The ultrasound bronchoscope 1290 may then be advanced through the airway wall into the parenchyma surrounding the airways. The access tool may then be removed and tools 1262, 1264 may be reinserted into ultrasound bronchoscope 1290 to engage the tissue at the target location for biopsy or treatment.

Registration, however, does not achieve a perfect match of the position of the patient's lungs and the 3D model. There are a number of reasons for this mismatch, typically called CT-to-body divergence. As an initial matter, traditional CT images are taken at full breath hold. That is, the patient is asked to expand their lungs to a maximum and hold that position while undergoing the imaging. This has the benefit of inflating the airways and increasing their visibility in the CT images and make it easier to generate a highly detailed 3D model. However, when performing the procedure, the patient is not at a full breath hold, rather they are typically sedated and experiencing tidal volume breathing. This results in a difference in shape and position of the airways in the lungs of the patient during the procedure as compared to during the CT imaging. As a result, even when the airways have been registered to the 3D model (e.g., using the airway sweep or another method) there will be differences between the relative positions of the airways or targets identified in the lungs in the model and the actual relative positions of the patient's airways and the target.

The ultrasound bronchoscope 1290 in accordance with the disclosure can address the CT-to-Body divergence. Ultrasound imaging of the target location, or tissue proximate the target location allows the system 1200 to visualize the target location. The ultrasound images captured by the ultrasound bronchoscope 1290 can be used to determine whether the end of the ultrasound bronchoscope is facing the target location and more particularly a target such as a lesion or tumor to be biopsied or treated. Any necessary adjustments to the position of the ultrasound bronchoscope 1290 can be made prior to advancement of tools 1262, 1264 through a working channel of the ultrasound bronchoscope 1290. Further, the ultrasound bronchoscope 1290 allows for visualization of the target tissue and the placement of the tools 1262, 1264 in the lesion or tumor as the tools are being inserted. This last mile navigation and position placement confirmation eliminates the need for other confirmatory measures such as fluoroscopy using a traditional fluoroscope 1220 which result in radiation dosing of both the patient and the clinical staff.

In a further aspect of the disclosure, the ultrasound bronchoscope 1290 is capable of transitioning between a radial ultrasound configuration to a linear ultrasound configuration. Utilization of the MEMS technology described herein in connection with CMUT and PMUT or with a polymer and printed circuit composed of polyvinylidene difluoride(PVDF) allows for such changes in configuration to be accomplished in software without necessarily changing the structural design of the ultrasound bronchoscope 1290. As an example, radial ultrasound imaging may be employed during the navigation from the trachea to the target location and upon achieving a determined distance from the target location linear ultrasound imaging can be employed to confirm the location of the lesion or tumor or other feature at the target location. Further linear ultrasound imaging can be used to confirm placement of a biopsy or treatment tool 1262, 1264.

It will be understood that various modifications may be made to the embodiments of the presently disclosed surgical systems and endoscopes. Therefore, the above description should not be construed as limiting, but merely as exemplifications of embodiments. Those skilled in the art will envision other modifications within the scope and spirit of the disclosure.

## Claims

1. A surgical system, comprising:
an endoscope defining an elongate body, the endoscope comprising;
an ultrasound sensor disposed on a distal portion of the elongate body;
a first channel defined through the elongate body of the endoscope, the first channel
defining a first aperture extending through the distal portion of the elongate body;
a second channel defined through the elongate body of the endoscope, the second
channel defining a second aperture extending through the distal portion of the elongate body; and
an optical waveguide disposed within the second channel;
a computing device, the computing device comprising;
a display screen; and
a processor including a memory associated therewith for storing a software application, which when executed, processes image data captured by the ultrasound sensor and displays a representation of a patient's anatomy on the display screen;
a vacuum source in fluid communication with the first channel, the vacuum source configured to cause a stone of a patient to be retained against the distal portion of the endoscope; and
a laser source in electromagnetic communication with the optical waveguide.

2. The surgical system according to claim 1, wherein the ultrasound sensor is a capacitive micromachined ultrasonic transducer.

3. The surgical system according to claim 1 or 2, wherein the ultrasound sensor is a piezoelectric based ultrasonic transducer.

4. The surgical system according to one of claims 1-3, further including a lens disposed on the distal portion of the elongate body of the endoscope and in electromagnetic communication with the optical waveguide, wherein, optionally, the lens defines a bevel thereon configured to direct propagation of energy generated by the laser at an angle relative to a longitudinal axis defined by the elongate body of the endoscope.

5. The surgical system according to claim 4, wherein the optical waveguide is rotatably supported within the second channel, wherein rotation of the optical waveguide relative to the lens causes the direction in which energy generated by the laser propagates therefrom.

6. The surgical system according to claim 4, wherein the lens is rotatably supported on the distal portion of the elongate body of the endoscope, wherein rotation of the lens relative to the optical waveguide causes the direction in which energy generated by the laser propagates therefrom.

7. The surgical system according to one of claims 1-6, wherein a portion of the first channel defines a protrusion thereon, the protrusion configured to collect fragments of a stone of a patient, wherein, optionally, the protrusion is an annular boss disposed on an inner surface of the first channel, and/or wherein, optionally, the protrusion is a protuberance disposed on an inner surface of the first channel.

8. An endoscope, comprising:
an elongate body defining a distal portion;
an ultrasound sensor disposed on the distal portion of the elongate body;
a first channel defined through the elongate body and defining a first aperture extending through the distal portion of the elongate body;
a second channel defined through the elongate body and defining a second aperture extending through the distal portion of the elongate body
a laser disposed within a portion of the elongate body;
an optical waveguide disposed within the second channel and in electromagnetic communication with the laser; and
a lens disposed on the distal portion of the elongate body and in electromagnetic communication with the optical waveguide, the lens configured to alter the direction in which energy generated by the laser propagates relative to the distal portion of the elongate body.

9. The endoscope according to claim 8, wherein the first channel is in fluid communication with a vacuum source, the vacuum source configured to cause a stone of a patient to be retained against the distal portion of the elongate body.

10. The endoscope according to claim 8 or 9, wherein the ultrasound sensor is a capacitive micromachined ultrasonic transducer.

11. The endoscope according to one of claims 8-10, wherein the ultrasound sensor is a piezoelectric based ultrasonic transducer.

12. The endoscope according to one of claims 8-11, wherein the lens defines a bevel thereon configured to direct propagation of energy generated by the laser at an angle relative to a longitudinal axis defined by the elongate body of the endoscope.

13. The endoscope according to claim 12, wherein the optical waveguide is rotatably supported within the second channel, wherein rotation of the optical waveguide relative to the lens causes the direction in which energy generated by the laser propagates therefrom.

14. The endoscope according to claim 12, wherein the lens is rotatably supported on the distal portion of the elongate body, wherein rotation of the lens relative to the optical waveguide causes the direction in which energy generated by the laser propagates therefrom.

15. The endoscope according to one of claims 8-14, wherein a portion of the first channel defines a protrusion thereon, the protrusion configured to collect fragments of a stone of a patient, wherein, optionally, the protrusion is an annular boss disposed on an inner surface of the first channel, and/or wherein, optionally, the protrusion is a protuberance disposed on an inner surface of the first channel.
